# EUROPEAN PATENT SPECIFICATION

(11) **EP 1 761 494 B1**
(45) Date of publication and mention of the grant of the patent: **21.04.2010**
(21) Application number: 05753676.5
(22) Date of filing: 20.06.2005
(51) Int. Cl.: C07D 211/58, C07D 401/06, C07D 409/06, C07D 413/06, C07D 417/06, A61K 31/4523, A61P 11/00, A61P 17/00, A61P 19/00, A61P 29/00, A61P 37/00

(54) **CHEMICAL COMPOUNDS I**
CHEMISCHE VERBINDUNGEN I
COMPOSES CHIMIQUES I

(30) Priority: 24.06.2004 SE 0401657
(43) Date of publication of application: 14.03.2007
(73) Proprietor: AstraZeneca AB, 151 85 Södertälje (SE)
(72) Inventor: TUCKER, Howard, Macclesfield, Cheshire SK10 5UE (GB)
(86) International application number: PCT/SE2005/000952
(87) International publication number: WO 2006/001751

(56) References cited:
- WO-A1-01/14333
- WO-A1-99/25686
- WO-A1-2004/056773
- WO-A2-2004/054974

## Description

The present invention relates to heterocyclic derivatives having pharmaceutical activity, to processes for preparing such derivatives, to pharmaceutical compositions comprising such derivatives and to the use of such derivatives as active therapeutic agents.

Pharmaceutically active piperidine derivatives are disclosed in WO03/042205. Pharmaceuticals need to be bioavailable and the compounds of the present invention show better levels of bioavailability, and greater selectivity (for example reduced muscarinic activity) than the compounds of WO03/042205.

Chemokines are chemotactic cytokines that are released by a wide variety of cells to attract macrophages, T cells, eosinophils, basophils and neutrophils to sites of inflammation and also play a rôle in the maturation of cells of the immune system. Chemokines play an important rôle in immune and inflammatory responses in various diseases and disorders, including asthma and allergic diseases, as well as autoimmune pathologies such as rheumatoid arthritis and atherosclerosis. These small secreted molecules are a growing superfamily of 8-14 kDa proteins characterised by a conserved four cysteine motif. The chemokine superfamily can be divided into two main groups exhibiting characteristic structural motifs, the Cys-X-Cys (C-X-C, or α) and Cys-Cys (C-C, or β) families. These are distinguished on the basis of a single amino acid insertion between the NH-proximal pair of cysteine residues and sequence similarity.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils such as human monocyte chemotactic proteins 1-3 (MCP-1, MCP-2 and MCP-3), RANTES (Regulated on Activation, Normal T Expressed and Secreted), eotaxin and the macrophage inflammatory proteins 1α and 1β (MIP-1α and MIP-1β).

Studies have demonstrated that the actions of the chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10, CXCR1, CXCR2, CXCR3 and CXCR4. These receptors represent good targets for drug development since agents which modulate these receptors would be useful in the treatment of disorders and diseases such as those mentioned above.

The CCR5 receptor is expressed on T-lymphocytes, monocytes, macrophages, dendritic cells, microglia and other cell types. These detect and respond to several chemokines, principally "regulated on activation normal T-cell expressed and secreted" (RANTES), macrophage inflammatory proteins (MIP) MIP-1α and MIP-1β and monocyte chemoattractant protein-2 (MCP-2).

This results in the recruitment of cells of the immune system to sites of disease. In many diseases it is the cells expressing CCR5 which contribute, directly or indirectly, to tissue damage. Consequently, inhibiting the recruitment of these cells is beneficial in a wide range of diseases.

CCR5 is also a co-receptor for HIV-1 and other viruses, allowing these viruses to enter cells. Blocking the receptor with a CCR5 antagonist or inducing receptor internalisation with a CCR5 agonist protects cells from viral infection.

The present invention provides compound *N*-(1-{(3*R*)-3-(3,5-difluorophenyl)-3-[1-(methylsulfonyl)piperidin-4-yl]propyl}piperidin-4-yl)-*N*-ethyl-2-[4-(methylsulfonyl)phenyl]-acetamide, or a pharmaceutically acceptable salt thereof.

Certain compounds of the present invention can exist in different isomeric forms (such as enantiomers, diastereomers, geometric isomers or tautomers). The present invention covers all such isomers and mixtures thereof in all proportions.

Suitable salts include acid addition salts such as a hydrochloride, hydrobromide, phosphate, acetate, fumarate, maleate, succinate, tartrate, citrate, oxalate, methanesulphonate or *p*-toluenesulphonate.

The compounds of the invention may exist as solvates (such as hydrates) and the present invention covers all such solvates.

In another aspect the present invention provides the compound: *N*-(1-{(3*R*)-3-(3,5-difluorophenyl)-3-[1-(methylsulfonyl)piperidin-4-yl]propyl}piperidin-4-yl)-*N*-ethyl-2-[4-(methylsulfonyl)phenyl]-acetamide; the succinate salt of *N*-(1-{(3*R*)-3-(3,5-difluorophenyl)-3-[1-(methylsulfonyl)piperidin-4-yl]propyl}piperidin-4-yl)-*N*-ethyl-2-[4-(methylsulfonyl)phenyl]acetamide; the fumarate salt of *N*-(1-{(3*R*)-3-(3,5-difluorophenyl)-3-[1-(methylsulfonyl)piperidin-4-yl]propyl}piperidin-4-yl)-*N*-ethyl-2-[4-(methylsulfonyl)phenyl]acetamide; or, *N*-(1-{(3*R*)-3-(3,5-difluorophenyl)-3-[1-(methylsulfonyl)piperidin-4-yl]propyl}piperidin-4-yl)-*N*-ethyl-2-[3-(methylsulfonyl)phenyl]acetamide.

The compounds listed in Table I illustrate the invention.

**TABLE I**

| | | | | | |
|---|---|---|---|---|---|
| Table I comprises compounds of formula (Ia). | | | | | |
| 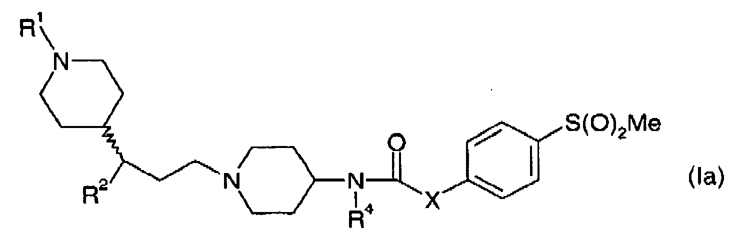 | | | | | |

| Compound No. | Stereo-chemistry | R¹ | R² | R⁴ | X |
|---|---|---|---|---|---|
| 1 | R | methanesulphonyl | 3,5-difluorophenyl | ethyl | CH₂ |
| 2 | R | methanesulphonyl | 3,5-difluorophenyl | ethyl | NHCH₂ |
| 3 | R | methanesulphonyl | 3-CF₃-phenyl | ethyl | CH₂ |
| 4 | R | methanesulphonyl | 3,5-difluorophenyl | cyclopropyl | CH₂ |
| 5 | R | methanesulphonyl | 3-fluoro-5-chlorophenyl | ethyl | CH₂ |
| 6 | R | methanesulphonyl | 3-fluorophenyl | ethyl | CH₂ |

In a further aspect the invention provides each individual compound listed in Table I.

The compounds of the invention can be prepared by using or modifying the preparative methodologies, Methods, Schemes or Examples of WO03/042205.

Thus, a compound of the invention can be prepared by reacting a compound of formula (II): 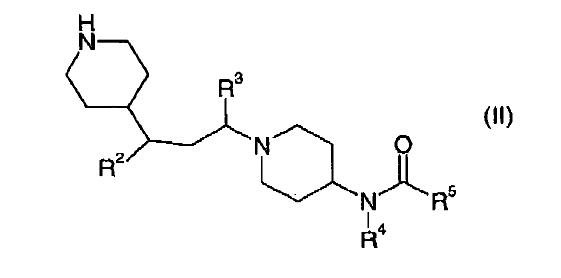 wherein R², R³, R⁴ and R⁵ are as defined above, with, depending on the compound of the invention it is desired to make:
a. an acid of formula R¹CO₂H in the presence of a suitable coupling agent (for example PyBrOP [bromo-tris-pyrrolidino-phosphonium hexafluorophosphate] or HATU [O-(7-azabenzotriazol-1-yl)-*N,N,N',N'*-tetramethyluronium hexafluorophosphate]) in the presence of a suitable base (such as a tri(C₁₋₆ alkyl)amine, for example diisopropylethylamine) in a suitable solvent (for example *N*-methylpyrrolidinone or a chlorinated solvent, such as dichloromethane) at room temperature (for example 10-30°C); or,
b. an acid chloride of formula R¹C(O)Cl or sulphonyl chloride of formula R¹S(O)₂Cl, in the presence of a suitable base (such as a tri(C₁₋₆ alkyl)amine, for example triethylamine or diisopropylethylamine) in a suitable solvent (for example a chlorinated solvent, such as dichloromethane) at room temperature (for example 10-30°C).

Alternatively, a compound of the invention can be prepared by coupling a compound of formula (III): 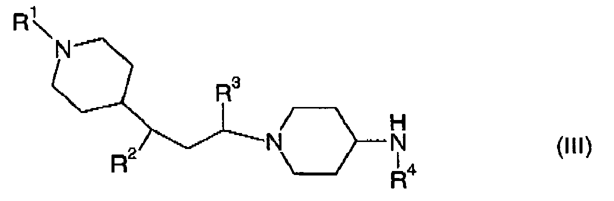 wherein R¹, R², R³ and R⁴ are as defined above, with:
a) an acid of formula R⁵CO₂H in the presence of a suitable coupling agent (for example PyBrOP or HATU) in the presence of a suitable base (such as a tri(C₁₋₆ alkyl)amine, for example diisopropylethylamine) in a suitable solvent (for example *N-*methylpyrrolidinone or a chlorinated solvent, such as dichloromethane) at room temperature (for example 10-30°C); or,
b) an acid chloride of formula R⁵C(O)Cl, in the presence of a suitable base (such as a tri(C₁₋₆ alkyl)amine, for example triethylamine or diisopropylethylamine) in a suitable solvent (for example a chlorinated solvent, such as dichloromethane) at room temperature (for example 10-30°C).

A compound of the invention can be prepared by reductive amination of a compound of formula (IV): 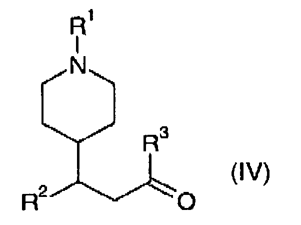 with a compound of formula (V): 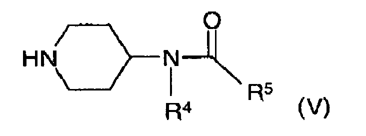 in the presence of NaBH(OAc)₃ (wherein Ac is C(O)CH₃) and acetic acid, in a suitable solvent (such as a C₁₋₆ aliphatic alcohol, for example ethanol) at room temperature (for example 10-30°C).

A compound of formula (V) can be prepared by removal of the protecting group (PG) from a compound of formula (VI). For example where PG is benzyloxylcarbonyl or benzyl removal may be effected by hydrogenation (for example hydrogen in the presence of palladium on carbon catalyst); where PG is *tert*-butyloxycarbonyl removal may be effected by treatment with acid (such as hydrochloric acid or trifluoroacetic acid).

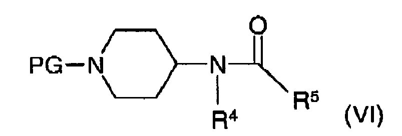

A compound of the invention can also be prepared by the alkylation of a compound of formula (V) with a compound of formula (VII): 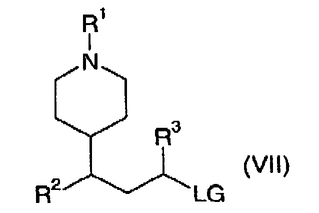 wherein R¹, R² and R³ are as defined above, and LG is a leaving group (such as halide, mesylate, tosylate or triflate); in the presence of a suitable base (such as potassium carbonate or a tertiary amine (for example Hünig's base or triethylamine)) in a suitable solvent (such as acetonitrile or THF) at room temperature (for example 10-30°C). Compounds of formula (VII) can be prepared by methods described, or by routine adaptation of methods described, in the patent or scientific literature.

In the processes described suitable protecting groups and details of processes for adding and removing such groups may be found in "Protective Groups in Organic Synthesis", 3rd Edition (1999) by Greene and Wuts.

The starting materials for these processes are either commercially available or can be prepared by literature methods, adapting literature methods or by following or the disclosure of WO03/042205.

In a still further aspect the invention provides processes for preparing the compounds of formula (I) or (Ia). Many of the intermediates in the processes are novel and these are provided as further features of the invention.

A compound of the invention, or a pharmaceutically acceptable salt thereof, can be used in the treatment of:
1. respiratory tract: obstructive diseases of the airways including: asthma, including bronchial, allergic, intrinsic, extrinsic, exercise-induced, drug-induced (including aspirin and NSAID-induced) and dust-induced asthma, both intermittent and persistent and of all severities, and other causes of airway hyper-responsiveness; chronic obstructive pulmonary disease (COPD); bronchitis, including infectious and eosinophilic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) and adenovirus;
2. bone and joints: arthritides associated with or including osteoarthritis/osteoarthrosis, both primary and secondary to, for example, congenital hip dysplasia; cervical and lumbar spondylitis, and low back and neck pain; rheumatoid arthritis and Still's disease; seronegative spondyloarthropathies including ankylosing spondylitis, psoriatic arthritis, reactive arthritis and undifferentiated spondarthropathy; septic arthritis and other infection-related arthopathies and bone disorders such as tuberculosis, including Potts' disease and Poncet's syndrome; acute and chronic crystal-induced synovitis including urate gout, calcium pyrophosphate deposition disease, and calcium apatite related tendon, bursal and synovial inflammation; Behcet's disease; primary and secondary Sjogren's syndrome; systemic sclerosis and limited scleroderma; systemic lupus erythematosus, mixed connective tissue disease, and undifferentiated connective tissue disease; inflammatory myopathies including dermatomyositits and polymyositis; polymalgia rheumatica; juvenile arthritis including idiopathic inflammatory arthritides of whatever joint distribution and associated syndromes, and rheumatic fever and its systemic complications; vasculitides including giant cell arteritis, Takayasu's arteritis, Churg-Strauss syndrome, polyarteritis nodosa, microscopic polyarteritis, and vasculitides associated with viral infection, hypersensitivity reactions, cryoglobulins, and paraproteins; low back pain; Familial Mediterranean fever, Muckle-Wells syndrome, and Familial Hibernian Fever, Kikuchi disease; drug-induced arthalgias, tendonititides, and myopathies;
3. pain and connective tissue remodelling of musculoskeletal disorders due to injury [for example sports injury] or disease: arthitides (for example rheumatoid arthritis, osteoarthritis, gout or crystal arthropathy), other joint disease (such as intervertebral disc degeneration or temporomandibular joint degeneration), bone remodelling disease (such as osteoporosis, Paget's disease or osteonecrosis), polychondritits, scleroderma, mixed connective tissue disorder, spondyloarthropathies or periodontal disease (such as periodontitis),
4. skin: psoriasis, atopic dermatitis, contact dermatitis or other eczematous dermatoses, and delayed-type hypersensitivity reactions; phyto- and photodermatitis; seborrhoeic dermatitis, dermatitis herpetiformis, lichen planus, lichen sclerosus et atrophica, pyoderma gangrenosum, skin sarcoid, discoid lupus erythematosus, pemphigus, pemphigoid, epidermolysis bullosa, urticaria, angioedema, vasculitides, toxic erythemas, cutaneous eosinophilias, alopecia areata, male-pattern baldness, Sweet's syndrome, Weber-Christian syndrome, erythema multiforme; cellulitis, both infective and non-infective; panniculitis;cutaneous lymphomas, non-melanoma skin cancer and other dysplastic lesions; drug-induced disorders including fixed drug eruptions;
5. eyes: blepharitis; conjunctivitis, including perennial and vernal allergic conjunctivitis; iritis; anterior and posterior uveitis; choroiditis; autoimmune; degenerative or inflammatory disorders affecting the retina; ophthalmitis including sympathetic ophthalmitis; sarcoidosis; infections including viral , fungal, and bacterial;
6. gastrointestinal tract: glossitis, gingivitis, periodontitis; oesophagitis, including reflux; eosinophilic gastro-enteritis, mastocytosis, Crohn's disease, colitis including ulcerative colitis, proctitis, pruritis ani; coeliac disease, irritable bowel syndrome, and food-related allergies which may have effects remote from the gut (for example migraine, rhinitis or eczema);
7. abdominal: hepatitis, including autoimmune, alcoholic and viral; fibrosis and cirrhosis of the liver; cholecystitis; pancreatitis, both acute and chronic;
8. genitourinary: nephritis including interstitial and glomerulonephritis; nephrotic syndrome; cystitis including acute and chronic (interstitial) cystitis and Hunner's ulcer; acute and chronic urethritis, prostatitis, epididymitis, oophoritis and salpingitis; vulvo-vaginitis; Peyronie's disease; erectile dysfunction (both male and female);
9. allograft rejection: acute and chronic following, for example, transplantation of kidney, heart, liver, lung, bone marrow, skin or cornea or following blood transfusion; or chronic graft versus host disease;
10. CNS: Alzheimer's disease and other dementing disorders including CJD and nvCJD; amyloidosis; multiple sclerosis and other demyelinating syndromes; cerebral atherosclerosis and vasculitis; temporal arthritis; myasthenia gravis; acute and chronic pain (acute, intermittent or persistent, whether of central or peripheral origin) including visceral pain, headache, migraine, trigeminal neuralgia, atypical facial pain, joint and bone pain, pain arising from cancer and tumor invasion, neuropathic pain syndromes including diabetic, post-herpetic, and HIV-associated neuropathies; neurosarcoidosis; central and peripheral nervous system complications of malignant, infectious or autoimmune processes;
11. other auto-immune and allergic disorders including Hashimoto's thyroiditis, Graves' disease, Addison's disease, diabetes mellitus, idiopathic thrombocytopaenic purpura, eosinophilic fasciitis, hyper-IgE syndrome, antiphospholipid syndrome;
12. other disorders with an inflammatory or immunological component including acquired immune deficiency syndrome (AIDS), leprosy, Sezary syndrome, and paraneoplastic syndromes;
13. cardiovascular: atherosclerosis, affecting the coronary and peripheral circulation; pericarditis; myocarditis , inflammatory and auto-immune cardiomyopathies including myocardial sarcoid; ischaemic reperfusion injuries; endocarditis, valvulitis, and aortitis including infective (for example syphilitic); vasculitides; disorders of the proximal and peripheral veins including phlebitis and thrombosis, including deep vein thrombosis and complications of varicose veins;
14. oncology: treatment of common cancers including prostate, breast, lung, ovarian, pancreatic, bowel and colon, stomach, skin and brain tumors and malignancies affecting the bone marrow (including the leukaemias) and lymphoproliferative systems, such as Hodgkin's and non-Hodgkin's lymphoma; including the prevention and treatment of metastatic disease and tumour recurrence, and paraneoplastic syndromes; or,
15. gastrointestinal tract: Coeliac disease, proctitis, eosinopilic gastro-enteritis, mastocytosis, Crohn's disease, ulcerative colitis, microscopic colitis, indeterminant colitis, irritable bowel disorder, irritable bowel syndrome, non-inflammatory diarrhea, food-related allergies which have effects remote from the gut, e.g., migraine, rhinitis and eczema; in a warm blooded animal, such as man.

The compounds of the invention have activity as pharmaceuticals, in particular as modulators (such as agonists, partial agonists, inverse agonists or antagonists) of chemokine receptor (for example CCR5) activity, and may be used in the treatment of autoimmune, inflammatory, proliferative or hyperproliferative diseases, or immunologically-mediated diseases (including rejection of transplanted organs or tissues and Acquired Immunodeficiency Syndrome (AIDS)).

The compounds of the present invention are also of value in inhibiting the entry of viruses (such as human immunodeficiency virus (HIV)) into target calls and, therefore, are of value in the prevention of infection by viruses (such as HIV), the treatment of infection by viruses (such as HIV) and the prevention and/or treatment of acquired immune deficiency syndrome (AIDS).

According to a further feature of the invention there is provided a compound of the formula (I) or (Ia), or a pharmaceutically acceptable salt thereof, for use in a method of treatment of a warm blooded animal (such as man) by therapy (including prophylaxis).

According to a further feature of the present invention there is provided a method for modulating chemokine receptor activity (for example CCR5 receptor activity) in a warm blooded animal, such as man, in need of such treatment, which comprises administering to said animal an effective amount of a compound of the present invention, or a pharmaceutically acceptable salt thereof.

The present invention also provides the use of a compound of the formula (I) or (Ia), or a pharmaceutically acceptable salt thereof, as a medicament, for example a medicament for the treatment of transplant rejection, respiratory disease, psoriasis or rheumatoid arthritis (for example rheumatoid arthritis). [Respiratory disease is, for example, COPD, asthma {such as bronchial, allergic, intrinsic, extrinsic or dust asthma, particularly chronic or inveterate asthma (for example late asthma or airways hyper-responsiveness)} or rhinitis {acute, allergic, atrophic rhinitis or chronic rhinitis including rhinitis caseosa, hypertrophic rhinitis rhinitis purulenta, rhinitis sicca or rhinitis medicamentosa; membranous rhinitis including croupous, fibrinous or pseudomembranous rhinitis or scrofoulous rhinitis; seasonal rhinitis including rhinitis nervosa (hay fever) or vasomotor rhinitis}; and is particularly asthma or rhinitis].

In another aspect the present invention provides the use of a compound of the formula (I) or (Ia), or a pharmaceutically acceptable salt thereof, in the manufacture of a medicament for use in therapy (for example modulating chemokine receptor activity (for example CCR5 receptor activity (such as rheumatoid arthritis)) in a warm blooded animal, such as man).

The invention also provides a compound of the formula (I) or (Ia), or a pharmaceutically acceptable salt thereof, for use as a medicament, for example a medicament for the treatment of rheumatoid arthritis.

In another aspect the present invention provides the use of a compound of the formula (I) or (Ia), or a pharmaceutically acceptable salt thereof, in the manufacture of a medicament for use in therapy (for example modulating chemokine receptor activity (for example CCR5 receptor activity (for example rheumatoid arthritis)) in a warm blooded animal, such as man).

The invention further provides the use of a compound of formula (I) or (Ia), or a pharmaceutically acceptable salt thereof, in the manufacture of a medicament for use in the treatment of:
1. respiratory tract: obstructive diseases of the airways including: asthma, including bronchial, allergic, intrinsic, extrinsic, exercise-induced, drug-induced (including aspirin and NSAID-induced) and dust-induced asthma, both intermittent and persistent and of all severities, and other causes of airway hyper-responsiveness; chronic obstructive pulmonary disease (COPD); bronchitis, including infectious and eosinophilic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) and adenovirus;
2. bone and joints; arthritides associated with or including osteoarthritis/osteoarthrosis, both primary and secondary to, for example, congenital hip dysplasia; cervical and lumbar spondylitis, and low back and neck pain; rheumatoid arthritis and Still's disease; seronegative spondyloarthropathies including ankylosing spondylitis, psoriatic arthritis, reactive arthritis and undifferentiated spondarthropathy; septic arthritis and other infection-related arthopathies and bone disorders such as tuberculosis, including Potts' disease and Poncet's syndrome; acute and chronic crystal-induced synovitis including urate gout, calcium pyrophosphate deposition disease, and calcium apatite related tendon, bursal and synovial inflammation; Behcet's disease; primary and secondary Sjogren's syndrome; systemic sclerosis and limited scleroderma; systemic lupus erythematosus, mixed connective tissue disease, and undifferentiated connective tissue disease; inflammatory myopathies including dermatomyositits and polymyositis; polymalgia rheumatica; juvenile arthritis including idiopathic inflammatory arthritides of whatever joint distribution and associated syndromes, and rheumatic fever and its systemic complications; vasculitides including giant cell arthritis, Takayasu's arteritis, Churg-Strauss syndrome, polyarteritis nodosa, microscopic polyarteritis, and vasculitides associated with viral infection, hypersensitivity reactions, cryoglobulins, and paraproteins; low back pain; Familial Mediterranean fever, Muckle-Wells syndromes, and Familial Hibernian Fever, Kikuchi disease; drug-induced arthalgias, tendonititides, and myopathies;
3. pain and connective tissue remodelling of musculoskeletal disorders due to injury [for example sports injury] or disease: arthitides (for example rheumatoid arthritis, osteoarthritis, gout or crystal arthropathy), other joint disease (such as intervertebral disc degeneration or temporomandibular joint degeneration), bone remodelling disease (such as osteoporosis, Paget's disease or osteonecrosis), polychondritits, scleroderma, mixed connective tissue disorder, spondyloarthropathies or periodontal disease (such as periodontitis);
4. skin: psoriasis, atopic dermatitis, contact dermatitis or other eczematous dermatoses, and delayed-type hypersensitivity reactions; phyto- and photodermatitis; seborrhoeic dermatitis, dermatitis herpetiformis, lichen planus, lichen sclerosus et atrophica, pyoderma gangrenosum, skin sarcoid, discoid lupus erythematosus, pemphigus, pemphigoid, epidermolysis bullosa, urticaria, angioedema, vasculitides, toxic erythemas, cutaneous eosinophilias, alopecia areata, male-pattern baldness, Sweet's syndrome, Weber-Christian syndrome, erythema multiforme; cellulitis, both infective and non-infective; panniculitis;cutaneous lymphomas, non-melanoma skin cancer and other dysplastic lesions; drug-induced disorders including fixed drug eruptions;
5. eyes: blepharitis; conjunctivitis, including perennial and vernal allergic conjunctivitis; iritis; anterior and posterior uveitis; choroiditis; autoimmune; degenerative or inflammatory disorders affecting the retina; ophthalmitis including sympathetic ophthalmitis; sarcoidosis; infections including viral, fungal, and bacterial;
6. gastrointestinal tract: glossitis, gingivitis, periodontitis; oesophagitis, including reflux; eosinophilic gastro-enteritis, mastocytosis, Crohn's disease, colitis including ulcerative colitis, proctitis, pruritis ani; coeliac disease, irritable bowel syndrome, and food-related allergies which may have effects remote from the gut (for example migraine, rhinitis or eczema);
7. abdominal; hepatitis, including autoimmune, alcoholic and viral; fibrosis and cirrhosis of the liver; cholecystitis; pancreatitis, both acute and chronic;
8. genitourinary: nephritis including interstitial and glomerulonephritis; nephrotic syndrome; cystitis including acute and chronic (interstitial) cystitis and Hunner's ulcer; acute and chronic urethritis, prostatitis, epididymitis, oophoritis and salpingitis; vulvo-vaginitis; Peyronie's disease; erectile dysfunction (both male and female);
9. allograft rejection: acute and chronic following, for example, transplantation of kidney, heart, liver, lung, bone marrow, skin or cornea or following blood transfusion; or chronic graft versus host disease;
10. CNS: Alzheimer's disease and other dementing disorders including CJD and nvCJD; amyloidosis; multiple sclerosis and other demyelinating syndromes; cerebral atherosclerosis and vasculitis; temporal arteritis; myasthenia gravis; acute and chronic pain (acute, intermittent or persistent, whether of central or peripheral origin) including visceral pain, headache, migraine, trigeminal neuralgia, atypical facial pain, joint and bone pain, pain arising from cancer and tumor invasion, neuropathic pain syndromes including diabetic, post-herpetic, and HIV-associated neuropathies; neurosarcoidosis; central and peripheral nervous system complications of malignant, infectious or autoimmune processes;
11. other auto-immune and allergic disorders including Hashimoto's thyroiditis, Graves' disease, Addison's disease, diabetes mellitus, idiopathic thrombocytopaenic purpura, eosinophilic fasciitis, hyper-IgE syndrome, antiphospholipid syndrome;
12. other disorders with an inflammatory or immunological component; including acquired immune deficiency syndrome (AIDS), leprosy, Sezary syndrome, and paraneoplastic syndromes;
13. cardiovascular: atherosclerosis, affecting the coronary and peripheral circulation; pericarditis; myocarditis, inflammatory and auto-immune cardiomyopathies including myocardial sarcoid; ischaemic reperfusion injuries; endocarditis, valvulitis, and aortitis including infective (for example syphilitic); vasculitides; disorders of the proximal and peripheral veins including phlebitis and thrombosis, including deep vein thrombosis and complications of varicose veins;
14. oncology: treatment of common cancers including prostate, breast, lung, ovarian, pancreatic, bowel and colon, stomach, skin and brain tumors and malignancies affecting the bone marrow (including the leukaemias) and lymphoproliferative systems, such as Hodgkin's and non-Hodgkin's lymphoma; including the prevention and treatment of metastatic disease and tumour recurrences, and paraneoplastic syndromes; or,
15. gastrointestinal tract: Coeliac disease, proctitis, eosinophilic gastro-enteritis, mastocytosis, Crohn's disease, ulcerative colitis, microscopic colitis, indeterminant colitis, irritable bowel disorder, irritable bowel syndrome, non-inflammatory diarrhea, food-related allergies which have effects remote from the gut, e.g., migraine, rhinitis and eczema; in a warm blooded animal, such as man.

In another aspect the invention further provides the use of a compound of formula (I) or (Ia), or a pharmaceutically acceptable salt thereof, in the manufacture of a medicament for use in the treatment of:
(1) (the respiratory tract) obstructive diseases of airways including: chronic obstructive pulmonary disease (COPD) (such as irreversible COPD); asthma {such as bronchial, allergic, intrinsic, extrinsic or dust asthma, particularly chronic or inveterate asthma (for example late asthma or airways hyper-responsiveness)}; bronchitis {such as eosinophilic bronchitis}; acute, allergic, atrophic rhinitis or chronic rhinitis including rhinitis caseosa, hypertrophic rhinitis, rhinitis purulenta, rhinitis sicca or rhinitis medicamentosa; membranous rhinitis including croupous, fibrinous or pseudomembranous rhinitis or scrofoulous rhinitis; seasonal rhinitis including rhinitis nervosa (hay fever) or vasomotor rhinitis; sarcoidosis; farmer's lung and related diseases; nasal polyposis; fibroid lung or idiopathic interstitial pneumonia;
(2) (bone and joints) arthrides including rheumatic, infectious, autoimmune, seronegative spondyloarthropathies (such as ankylosing spondylitis, psoriatic arthritis or Reiter's disease), Behçet's disease, Sjogren's syndrome or systemic sclerosis;
(3) (skin and eyes) psoriasis, atopic dermatitis, contact dermatitis or other eczmatous dermitides, seborrhoetic dermatitis, Lichen planus, Phemphigus, bullous Phemphigus, Epidermolysis bullosa, urticaria, angiodermas, vasculitides erythemas, cutaneous eosinophilias, uveitis, Alopecia areata or vernal conjunctivitis;
(4) (gastrointestinal tract) Coeliac disease, proctitis, eosinophilic gastro-enteritis, mastocytosis, Crohn's disease, ulcerative colitis, irritable bowel disease or food-related allergies which have effects remote from the gut (for example migraine, rhinitis or eczema);
(5) (Allograft rejection) acute and chronic following, for example, transplantation of kidney, heart, liver, lung, bone marrow, skin or cornea; or chronic graft versus host disease; and/or
(6) (other tissues or diseases) Alzheimer's disease, multiple sclerosis, atherosclerosis, Acquired Immunodeficiency Syndrome (AIDS), Lupus disorders (such as lupus erythematosus or systemic lupus), erythematosus, Hashimoto's thyroiditis, myasthenia gravis, type I diabetes, nephrotic syndrome, eosinophilia fascitis, hyper IgE syndrome, leprosy (such as lepromatous leprosy), Peridontal disease, Sezary syndrome, idiopathic thrombocytopenia pupura or disorders of the menstrual cycle;
in a warm blooded animal, such as man.

The present invention further provides a method of treating a chemokine mediated disease state (for example a CCR5 mediated disease state) in a warm blooded animal, such as man, which comprises administering to a mammal in need of such treatment an effective amount of a compound of formula (I) or (Ia), or a pharmaceutically acceptable salt thereof.

In order to use a compound of the invention, or a pharmaceutically acceptable salt thereof, for the therapeutic treatment of a warm blooded animal, such as man, in particular modulating chemokine receptor (for example CCR5 receptor) activity, said ingredient is normally formulated in accordance with standard pharmaceutical practice as a pharmaceutical composition.

Therefore in another aspect the present invention provides a pharmaceutical composition which comprises a compound of the formula (I) or (Ia), or a pharmaceutically acceptable salt thereof (active ingredient), and a pharmaceutically acceptable adjuvant, diluent or carrier. In a further aspect the present invention provides a process for the preparation of said composition which comprises mixing active ingredient with a pharmaceutically acceptable adjuvant, diluent or carrier. Depending on the mode of administration, the pharmaceutical composition will comprise from 0.05 to 99 %w (per cent by weight), such as from 0.05 to 80 %w, for example from 0.10 to 70 %w (such as from 0.10 to 50 %w) of active ingredient, all percentages by weight being based on total composition.

The pharmaceutical compositions of this invention may be administered in standard manner for the disease condition that it is desired to treat, for example by topical (such as to the lung and/or airways or to the skin), oral, rectal or parenteral administration. For these purposes the compounds of this invention may be formulated by means known in the art into the form of, for example, aerosols, dry powder formulations, tablets, capsules, syrups, powders, granules, aqueous or oily solutions or suspensions, (lipid) emulsions, dispersible powders, suppositories, ointments, creams, drops and sterile injectable aqueous or oily solutions or suspensions.

A suitable pharmaceutical composition of this invention is one suitable for oral administration in unit dosage form, for example a tablet or capsule which contains between 0.1mg and 1g of active ingredient.

In another aspect a pharmaceutical composition of the invention is one suitable for intravenous, subcutaneous or intramuscular injection.

Each patient may receive, for example, an intravenous, subcutaneous or intramuscular dose of 0.01mgkg⁻¹ to 100mgkg⁻¹ of the compound, for example in the range of 0.1mgkg⁻¹ to 20mgkg⁻¹ of this invention, the composition being administered 1 to 4 times per day. The intravenous, subcutaneous and intramuscular dose may be given by means of a bolus injection. Alternatively the intravenous dose may be given by continuous infusion over a period of time. Alternatively each patient will receive a daily oral dose which is approximately equivalent to the daily parenteral dose, the composition being administered 1 to 4 times per day.

The following illustrate representative pharmaceutical dosage forms containing the compound of formula (I) or (Ia), or a pharmaceutically acceptable salt thereof (hereafter Compound X), for therapeutic or prophylactic use in humans:
(a)

| Tablet I | mg/tablet |
|---|---|
| Compound X | 100 |
| Lactose Ph.Eur. | 179 |
| Croscarmellose sodium | 12.0 |
| Polyvinylpyrrolidone | 6 |
| Magnesium stearate | 3.0 |

(b)

| Tablet II | mg/tablet |
|---|---|
| Compound X | 50 |
| Lactose Ph.Eur. | 229 |
| Croscarmellose sodium | 12.0 |
| Polyvinylpyrrolidone | 6 |
| Magnesium stearate | 3.0 |

(c)

| Tablet III | mg/tablet |
|---|---|
| Compound X | 1.0 |
| Lactose Ph.Eur. | 92 |
| Croscarmellose sodium | 4.0 |
| Polyvinylpyrrolidone | 2-0 |
| Magnesium stearate | 1.0 |

(d)

| Capsule | mg/capsule |
|---|---|
| Compound X | 10 |
| Lactose Ph.Eur. | 389 |
| Croscarmellose sodium | 100 |
| Magnesium stearate | 1.0 |

(e)

| Injection I | (50 mg/ml) |
|---|---|
| Compound X | 5.0% w/v |
| Isotonic aqueous solution | to 100% |

Buffers, pharmaceutically-acceptable cosolvents such as polyethylene glycol, polypropylene glycol, glycerol or ethanol or complexing agents such as hydroxy-propyl β-cyclodextrin may be used to aid formulation.

The above formulations may be obtained by conventional procedures well known in the pharmaceutical art. The tablets (a)-(c) may be enteric coated by conventional means, for example to provide a coating of cellulose acetate phthalate.

The invention further relates to a combination therapy wherein a compound of the invention, or a pharmaceutically acceptable salt thereof, or a pharmaceutical composition or formulation comprising a compound of the invention, is administered concurrently or sequentially or as a combined preparation with another therapeutic agent or agents, for the treatment of one or more of the conditions listed.

In particular, for the treatment of the inflammatory diseases such as (but not restricted to) rheumatoid arthritis, osteoarthritis, asthma, allergic rhinitis, chronic obstructive pulmonary disease (COPD), psoriasis, and inflammatory bowel disease, the compounds of the invention may be combined with agents listed below.

Non-steroidal anti-inflammatory agents (hereinafter NSAIDs) including non-selective cyclo-oxygenase COX-1 / COX-2 inhibitors whether applied topically or systemically (such as piroxicam, diclofenac, propionic acids such as naproxen, flurbiprofen, fenoprofen, ketoprofen and ibuprofen, fenamates such as mefenamic acid, indomethacin, sulindac, azapropazone, pyrazolones such as phenylbutazone, salicylates such as aspirin); selective COX-2 inhibitors (such as meloxicam, celecoxib, rofecoxib, valdecoxib, lumarocoxib, parecoxib and etoricoxib); cyclo-oxygenase inhibiting nitric oxide donors (CINODs); glucocorticosteroids (whether administered by topical, oral, intramuscular, intravenous, or intra-articular routes); methotrexate; leflunomide; hydroxychloroquine; d-penicillamine; auranofin or other parenteral or oral gold preparations; analgesics; diacerein; intra-articular therapies such as hyaluronic acid derivatives; and nutritional supplements such as glucosamine.

The present invention still further relates to the combination of a compound of the invention, or a pharmaceutically acceptable salt thereof, together with a cytokine or agonist or antagonist of cytokine function, (including agents which act on cytokine signalling pathways such as modulators of the SOCS system) including alpha-, beta-, and gamma-interferons; insulin-like growth factor type I (IGF-1); interleukins (IL) including IL1 to 17, and interleukin antagonists or inhibitors such as anakinra; tumour necrosis factor alpha (TNF-α) inhibitors such as anti-TNF monoclonal antibodies (for example infliximab; adalimumab, and CDP-870) and TNF receptor antagonists including immunoglobulin molecules (such as etanercept) and low-molecular-weight agents such as pentoxyfylline.

In addition the invention relates to a combination of a compound of the invention, or a pharmaceutically acceptable salt thereof, with a monoclonal antibody targeting B-Lymphocytes (such as CD20 (rituximab), MRA-aIL16R and T-Lymphocytes, CTLA4-1g, HuMax Il-15).

The present invention still further relates to the combination of a compound of the invention, or a pharmaceutically acceptable salt thereof, with a modulator of chemokine receptor function such as an antagonist of CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX₃CR1 for the C-X₃-C family.

The present invention further relates to the combination of a compound of the invention, or a pharmaceutically acceptable salt thereof, with an inhibitor of matrix metalloprotease (MMPs), i.e., the stromelysins, the collagenases, and the gelatinases, as well as aggrecanase; for example collagenase-1 (MMP-1), collagenase-2 (MMP-8), collagenase-3 (MMP-13), stromelysin-1 (MMP-3), stromelysin-2 (MMP-10), and stromelysin-3 (MMP-11) and MMP-9 and MMP-12, including agents such as doxycycline.

The present invention still further relates to the combination of a compound of the invention, or a pharmaceutically acceptable salt thereof, and a leukotriene biosynthesis inhibitor, 5-lipoxygenase (5-LO) inhibitor or 5-lipoxygenase activating protein (FLAP) antagonist such as; zileuton; ABT-761; fenleuton; tepoxalin; Abbott-79175; Abbot-85761; a N-(5-substituted)-thiophene-2-alkylsulfonamide; 2,6-di-tert-butylphenolhydrazones; a methoxytetrahydropyrans such as Zeneca ZD-2138; the compound SB-210661; a pyridinyl-substituted 2-cyanonaphthalene compound such as L-739,010; a 2-cyanoquinoline compound such as L-746,530; or an indole or quinoline compound such as MK-591, MK-886, and BAY x 1005.

The present invention further relates to the combination of a compound of the invention, or a pharmaceutically acceptable salt thereof, and a receptor antagonist for leukotrienes (LT) B4, LTC4, LTD4, and LTE4. selected from the group consisting of the phenothiazin-3-1s such as L-651,392; amidino compounds such as CGS-25019c; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY x 7195.

The present invention still further relates to the combination of a compound of the invention, or a pharmaceutically acceptable salt thereof, and a phosphodiesterase (PDE) inhibitor such as a methylxanthanine including theophylline and aminophylline; a selective PDE isoenzyme inhibitor including a PDE4 inhibitor an inhibitor of the isoform PDE4D, or an inhibitor of PDE5.

The present invention further relates to the combination of a compound of the invention, or a pharmaceutically acceptable salt thereof, and a histamine type 1 receptor antagonist such as cetirizine, loratadine, desloratadine, fexofenadine, acrivastine, terfenadine, astemizole, azelastine, levocabastine, chlorpheniramine, promethazine, cyclizine, or mizolastine; applied orally, topically or parenterally.

The present invention still further relates to the combination of a compound of the invention, or a pharmaceutically acceptable salt thereof, and a proton pump inhibitor (such as omeprazole) or a gastroprotective histamine type 2 receptor antagonist.

The present invention further relates to the combination of a compound of the invention, or a pharmaceutically acceptable salt thereof, and an antagonist of the histamine type 4 receptor.

The present invention still further relates to the combination of a compound of the invention, or a pharmaceutically acceptable salt thereof, and an alpha-1/alpha-2 adrenoceptor agonist vasoconstrictor sympathomimetic agent, such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride or ethylnorepinephrine hydrochloride.

The present invention further relates to the combination of a compound of the invention, or a pharmaceutically acceptable salt thereof, and an anticholinergic agents including muscarinic receptor (M1, M2, and M3) antagonist such as atropine, hyoscine, glycopyrrrolate, ipratropium bromide, tiotropium bromide, oxitropium bromide, pirenzepine or telenzepine.

The present invention still further relates to the combination of a compound of the invention, or a pharmaceutically acceptable salt thereof, and a beta-adrenoceptor agonist (including beta receptor subtypes 1-4) such as isoprenaline, salbutamol, formoterol, salmeterol, terbutaline, orciprenaline, bitolterol mesylate, or pirbuterol, or a chiral enantiomer thereof.

The present invention further relates to the combination of a compound of the invention, or a pharmaceutically acceptable salt thereof, and a chromone, such as sodium cromoglycate or nedocromil sodium.

The present invention still further relates to the combination of a compound of the invention, or a pharmaceutically acceptable salt thereof, with a glucocorticoid, such as flunisolide, triamcinolone acetonide, beclomethasone dipropionate, budesonide, fluticasone propionate, ciclesonide or mometasone furoate.

The present invention further relates to the combination of a compound of the invention, or a pharmaceutically acceptable salt thereof, with an agent that modulates a nuclear hormone receptor such as PPARs.

The present invention still further relates to the combination of a compound of the invention, or a pharmaceutically acceptable salt thereof, together with an immunoglobulin (Ig) or Ig preparation or an antagonist or antibody modulating Ig function such as anti-IgE (for example omalizumab).

The present invention further relates to the combination of a compound of the invention, or a pharmaceutically acceptable salt thereof, and another systemic or topically-applied anti-inflammatory agent, such as thalidomide or a derivative thereof, a retinoid, dithranol or calcipotriol.

The present invention still further relates to the combination of a compound of the invention, or a pharmaceutically acceptable salt thereof, and combinations of aminosalicylates and sulfapyridine such as sulfasalazine, mesalazine, balsalazide, and olsalazine; and immunomodulatory agents such as the thiopurines, and corticosteroids such as budesonide.

The present invention further relates to the combination of a compound of the invention, or a pharmaceutically acceptable salt thereof, together with an antibacterial agent such as a penicillin derivative, a tetracycline, a macrolide, a beta-lactam, a fluoroquinolone, metronidazole, an inhaled aminoglycoside; an antiviral agent including acyclovir, famciclovir, valaciclovir, ganciclovir, cidofovir, amantadine, rimantadine, ribavirin, zanamavir and oseltamavir; a protease inhibitor such as indinavir, nelfinavir, ritonavir, and saquinavir; a nucleoside reverse transcriptase inhibitor such as didanosine, lamivudine, stavudine, zalcitabine or zidovudine; or a non-nucleoside reverse transcriptase inhibitor such as nevirapine or efavirenz.

The present invention still further relates to the combination of a compound of the invention, or a pharmaceutically acceptable salt thereof, and a cardiovascular agent such as a calcium channel blocker, a beta-adrenoceptor blocker, an angiotensin-converting enzyme (ACE) inhibitor, an angiotensin-2 receptor antagonist; a lipid lowering agent such as a statin or a fibrate; a modulator of blood cell morphology such as pentoxyfylline; thrombolytic, or an anticoagulant such as a platelet aggregation inhibitor.

The present invention further relates to the combination of a compound of the invention, or a pharmaceutically acceptable salt thereof, and a CNS agent such as an antidepressant (such as sertraline), an anti-Parkinsonian drug (such as deprenyl, L-dopa, ropinirole, pramipexole, a MAOB inhibitor such as selegine and rasagiline, a comP inhibitor such as tasmar, an A-2 inhibitor, a dopamine reuptake inhibitor, an NMDA antagonist, a nicotine agonist, a dopamine agonist or an inhibitor of neuronal nitric oxide synthase), or an anti-Alzheimer's drug such as donepezil, rivastigmine, tacrine, a COX-2 inhibitor, propentofylline or metrifonate.

The present invention still further relates to the combination of a compound of the invention, or a pharmaceutically acceptable salt thereof, and an agent for the treatment of acute or chronic pain, such as a centrally or peripherally-acting analgesic (for example an opioid or derivative thereof), carbamazepine, phenytoin, sodium valproate, amitryptiline or other anti-depressant agent-s, paracetamol, or a non-steroidal anti-inflammatory agent.

The present invention further relates to the combination of a compound of the invention, or a pharmaceutically acceptable salt thereof, together with a parenterally or topically-applied (including inhaled) local anaesthetic agent such as lignocaine or a derivative thereof.

A compound of the present invention, or a pharmaceutically acceptable salt thereof, can also be used in combination with an anti-osteoporosis agent including a hormonal agent such as raloxifene, or a biphosphonate such as alendronate.

The present invention still further relates to the combination of a compound of the invention, or a pharmaceutically acceptable salt thereof, together with a: (i) tryptase inhibitor; (ii) platelet activating factor (PAF) antagonist; (iii) interleukin converting enzyme (ICE) inhibitor; (iv) IMPDH inhibitor; (v) adhesion molecule inhibitors including VLA-4 antagonist; (vi) cathepsin; (vii) kinase inhibitor such as an inhibitor of tyrosine kinase (such as Btk, Itk, Jak3 or MAP, for example Gefitinib or Imatinib mesylate), a serine / threonine kinase (such as an inhibitor of a MAP kinase such as p38, JNK, protein kinase A, B or C, or IKK), or a kinase involved in cell cycle regulation (such as a cylin dependent kinase); (viii) glucose-6 phosphate dehydrogenase inhibitor; (ix) kinin-B.sub1. - or B.sub2. -receptor antagonist; (x) anti-gout agent, for example colchicine; (xi) xanthine oxidase inhibitor, for example allopurinol; (xii) uricosuric agent, for example probenecid, sulfinpyrazone or benzbromarone; (xiii) growth hormone secretagogue; (xiv) transforming growth factor (TGFβ); (xv) platelet-derived growth factor (PDGF); (xvi) fibroblast growth factor for example basic fibroblast growth factor (bFGF); (xvii) granulocyte macrophage colony stimulating factor (GM-CSF); (xviii) capsaicin cream; (xix) tachykinin NK.sub1. or NK.sub3. receptor antagonist such as NKP-608C, SB-233412 (talnetant) or D-4418; (xx) elastase inhibitor such as UT-77 or ZD-0892; (xxi) TNF-alpha converting enzyme inhibitor (TACE); (xxii) induced nitric oxide synthase (iNOS) inhibitor; (xxiii) chemoattractant receptor-homologous molecule expressed on TH2 cells, (such as a CRTH2 antagonist); (xxiv) inhibitor of P38; (xxv) agent modulating the function of Toll-like receptors (TLR), (xxvi) agent modulating the activity of purinergic receptors such as P2X7; or (xxvii) inhibitor of transcription factor activation such as NFkB, API, or STATS.

A compound of the invention, or a pharmaceutically acceptable salt thereof, can also be used in combination with an existing therapeutic agent for the treatment of cancer, for example suitable agents include:
(i) an antiproliferative/antineoplastic drug or a combination thereof, as used in medical oncology, such as an alkylating agent (for example cis-platin, carboplatin, cyclophosphamide, nitrogen mustard, melphalan, chlorambucil, busulphan or a nitrosourea); an antimetabolite (for example an antifolate such as a fluoropyrimidine like 5-fluorouracil or tegafur, raltitrexed, methotrexate, cytosine arabinoside, hydroxyurea, gemcitabine or paclitaxel); an antitumour antibiotic (for example an anthracycline such as adriamycin, bleomycin, doxorubicin, daunomycin, epirubicin, idarubicin, mitomycin-C, dactinomycin or mithramycin); an antimitotic agent (for example a vinca alkaloid such as vincristine, vinblastine, vindesine or vinorelbine, or a taxoid such as taxol or taxotere); or a topoisomerase inhibitor (for example an epipodophyllotoxin such as etoposide, teniposide, amsacrine, topotecan or a camptothecin);
(ii) a cytostatic agent such as an antioestrogen (for example tamoxifen, toremifene, raloxifene, droloxifene or iodoxyfene), an oestrogen receptor down regulator (for example fulvestrant), an antiandrogen (for example bicalutamide, flutamide, nilutamide or cyproterone acetate), a LHRH antagonist or LHRH agonist (for example goserelin, leuprorelin or buserelin), a progestogen (for example megestrol acetate), an aromatase inhibitor (for example as anastrozole, letrozole, vorazole or exemestane) or an inhibitor of 5α-reductase such as finasteride;
(iii) an agent which inhibits cancer cell invasion (for example a metalloproteinase inhibitor like marimastat or an inhibitor of urokinase plasminogen activator receptor function);
(iv) an inhibitor of growth factor function, for example: a growth factor antibody (for example the anti-erbb2 antibody trastuzumab, or the anti-erbb1 antibody cetuximab [C225]), a farnesyl transferase inhibitor, a tyrosine kinase inhibitor or a serine/threonine kinase inhibitor, an inhibitor of the epidermal growth factor family (for example an EGFR family tyrosine kinase inhibitor such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) or 6-acrylamido-N-(3-chloro-4-fluorophenyl)-7-(3-morpholinopropoxy)quinazolin-4-amine (CI 1033)), an inhibitor of the platelet-derived growth factor family, or an inhibitor of the hepatocyte growth factor family;
(v) an antiangiogenic agent such as one which inhibits the effects of vascular endothelial growth factor (for example the anti-vascular endothelial cell growth factor antibody bevacizumab, a compound disclosed in WO 97/22596, WO 97/30035, WO 97/32856 or WO 98/13354), or a compound that works by another mechanism (for example linomide, an inhibitor of integrin αvβ3 function or an angiostatin);
(vi) a vascular damaging agent such as combretastatin A4, or a compound disclosed in WO 99/02166, WO 00/40529, WO 00/41669, WO 01/92224, WO 02/04434 or WO 02/08213;
(vii) an agent used in antisense therapy, for example one directed to one of the targets listed above, such as ISIS 2503, an anti-ras antisense;
(viii) an agent used in a gene therapy approach, for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy;
(ix) an agent used in an immunotherapeutic approach, for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies; or
(x) a compound useful in the treatment of AIDS and/or HIV infection for example: an agent which prevents or inhibits the viral protein gp120 from engaging host cell CD4 {such as soluble CD4 (recombinant); an anti-CD4 antibody (or modified / recombinant antibody) for example PRO542; an anti-group 120 antibody (or modified / recombinant antibody); or another agent which interferes with the binding of group120 to CD4 for example BMS806}; an agent which prevents binding to a chemokine receptor, other than CCR5, used by the HIV virus {such as a CXCR4 agonist or antagonist or an anti-CXCR4 antibody}; a compound which interferes in the fusion between the HIV viral envelope and a cell membrane {such as an anti-group 41 antibody; enfuvirtide (T-20) or T-1249}; an inhibitor of DC-SIGN (also known as CD209) {such as an anti-DC-SIGN antibody or an inhibitor of DC-SIGN binding}; a nucleoside/nucleotide analogue reverse transciptase inhibitor {for example zidovudine (AZT), nevirapine, didanosine (ddI), zalcitabine (ddC), stavudine (d4T), lamivudine (3TC), abacavir, adefovir or tenofovir (for example as free base or as disoproxil fumarate)}; a non-nucleoside reverse transciptase inhibitor {for example nevirapine, delavirdine or efavirenz}; a protease inhibitor {for example ritonavir, indinavir, saquinavir (for example as free base or as mesylate salt), nelfinavir (for example as free base or as mesylate salt), amprenavir, lopinavir or atazanavir (for example as free base or as sulphate salt)}; a ribonucleotide reductase inhibitor {for example hydroxyurea}; or an antiretroviral {for example emtricitabine}.

The invention will now be illustrated by the following non-limiting Examples in which, unless stated otherwise:
(i) temperatures are given in degrees Celsius (°C); operations were carried out at room or ambient temperature, that is, at a temperature in the range of 18-25°C;
(ii) organic solutions were dried over anhydrous magnesium sulfate; evaporation of solvent was carried out using a rotary evaporator under reduced pressure (600-4000 Pascals; 4.5-30 mm Hg) with a bath temperature of up to 60°C;
(iii) chromatography unless otherwise stated means flash chromatography on silica gel; thin layer chromatography (TLC) was carried out on silica gel plates; where a "Bond Elut" column is referred to, this means a column containing 10g or 20g of silica of 40 micron particle size, the silica being contained in a 60ml disposable syringe and supported by a porous disc, obtained from Varian, Harbor City, California, USA under the name "Mega Bond Elut SI". Where an "Isolute™ SCX column" is referred to, this means a column containing benzenesulphonic acid (non-endcapped) obtained from International Sorbent Technology Ltd., 1st House, Duffryn Industial Estate, Ystrad Mynach, Hengoed, Mid Glamorgan, UK. Where "Argonaut™ PS-*tris*-amine scavenger resin" is referred to, this means a *tris*-(2-aminoethyl)amine polystyrene resin obtained from Argonaut Technologies Inc., 887 Industrial Road, Suite G, San Carlos, California, USA.
(iv) in general, the course of reactions was followed by TLC and reaction times are given for illustration only;
(v) yields, when given, are for illustration only and are not necessarily those which can be obtained by diligent process development; preparations were repeated if more material was required;
(vi) when given, ¹H NMR data is quoted and is in the form of delta values for major diagnostic protons, given in parts per million (ppm) relative to tetramethylsilane (TMS) as an internal standard, determined at 300 MHz using perdeuterio DMSO (CD₃SOCD₃) as the solvent unless otherwise stated; coupling constants (J) are given in Hz;
(vii) chemical symbols have their usual meanings; SI units and symbols are used;
(viii) solvent ratios are given in percentage by volume;
(ix) mass spectra (MS) were run with an electron energy of 70 electron volts in the chemical ionisation (APCI) mode using a direct exposure probe; where indicated ionisation was effected by electrospray (ES); where values for m/z are given, generally only ions which indicate the parent mass are reported, and unless otherwise stated the mass ion quoted is the positive mass ion - (M+H)⁺;
(x) LCMS characterisation was performed using a pair of Gilson 306 pumps with Gilson 233 XL sampler and Waters ZMD4000 mass spectrometer. The LC comprised water symmetry 4.6x50 column C18 with 5 micron particle size. The eluents were: A, water with 0.05% formic acid and B, acetonitrile with 0.05% formic acid. The eluent gradient went from 95% A to 95% B in 6 minutes. Where indicated ionisation was effected by electrospray (ES); where values for m/z are given, generally only ions which indicate the parent mass are reported, and unless otherwise stated the mass ion quoted is the positive mass ion - (M+H)⁺ and
(xi) the following abbreviations are used:

| | |
|---|---|
| DMF | *N,N*-dimethylformamide; |
| m.pt. | melting point |
| TMEDA | *N,N,N',N'*-tetramethylethylenediamine; |
| THF | tetrahydrofuran. |

### EXAMPLE 1

This Example illustrates the preparation of *N*-(1-{(3*R*)-3-(3,5-difluorophenyl)-3-[1-(methylsulfonyl)piperidin-4-yl]propyl}piperidin-4-yl)-*N*-ethyl-2-[4-(methylsulfonyl)phenyl]-acetamide (Compound 1 of Table I)

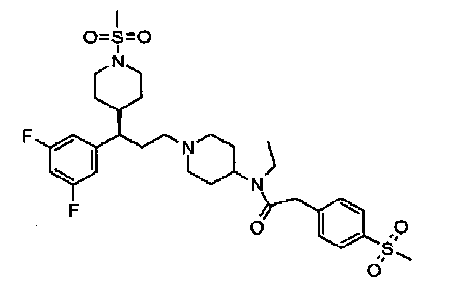

Sodium triacetoxyborohydride (2.5g) was added to a solution of (3R)-3-(3,5-difluorophenyl)-3-[1-(methylsulfonyl)piperidin-4-yl]propanal (1.98g) and *N*-ethyl-2-[4-(methylsulfonyl)phenyl]-*N*-piperidin-4-ylacetamide (1.94g) in dichloromethane (100 ml) and the mixture was stirred for 2 hours, then washed with 2M NaOH (2x100 ml) and dried. The organics were poured on to a 50g SCX2 cartridge and eluted with methanol (6x50 ml) and 1M methanolic ammonia (7x50 ml). The combined methanolic ammonia washings were evaporated to dryness to give the title compound as a white foam, yield 2.6g. NMR (CDCl₃): 1.2-2.1 (m, 19H), 2.3-2.7 (m, 3H), 2.75 (s, 3H), 2.8-2.9 (m, 2H), 3.0 (s, 3H), 3.3 (q, 2H), 3.7-3.9 (m, 4H), 6.65 (m, 3H), 7.4 (m, 2H), 7.9 (d, 2H).

### EXAMPLE 2

This Example illustrates the preparation of the succinate salt of *N*-(1-{(3*R*)-3-(3,5-difluorophenyl)-3-[1-(methylsulfonyl)piperidin-4-yl]propyl}piperidin-4-yl)-*N*-ethyl-2-[4-(methylsulfonyl)phenyl]acetamide.

A hot solution of succinic acid (59 mgs) in ethanol (3 ml) was added to a hot solution of *N-*(1-{(3*R*)-3-(3,5-difluorophenyl)-3-[1-(methylsulfonyl)piperidin-4-yl]propyl}piperidin-4-yl)-*N*-ethyl-2-[4-(methylsulfonyl)phenyl]acetamide (640 mgs) in ethanol (16 ml) and the mixture was allowed to cool. Alter 24 hours the mixture was triturated using a spatula and allowed to stand for an additional 24 hours. The solid was filtered and dried, m.pt. 175-175.5 °C. This solid was recyrstallized from ethanol (14 ml) to give the title compound, m.pt. 177-177.5°C.

### EXAMPLE 3

This Example illustrates the preparation of the fumarate salt of *N*-(1-{(3*R*)-3-(3,5-difluorophenyl)-3-[1-(methylsulfonyl)piperidin-4-yl]propyl}piperidin-4-yl)-*N*-ethyl-2-[4-(methylsulfonyl)phenyl]acetamide.

A hot solution of fumaric acid (55 mgs) in methanol (3 ml) was added to a hot solution of *N*-(1-{(3*R*)-3-(3,5-difluorophenyl)-3-[1-(methylsulfonyl)piperidin-4-yl]propyl}piperidin-4-yl)-*N*-ethyl-2-[4-(methylsulfonyl)phenyl]acetamide (300 mgs) in methanol (4 ml) and the mixture allowed to cool. The mixture was then cooled in an ice bath and finally allowed to stand in a refrigerator for 14 hours. The solid was filtered and dried at 60 °C under vacuum for 2 hours m.pt. 164-166.5°C.

### EXAMPLE 4

This Example illustrates the preparation of *N*-(1-{(3R)-3-(3,5-difluorophenyl)-3-[1-(methylsulfonyl)piperidin-4-yl]propyl}piperidin-4-yl)-*N*-ethyl-2-[3-(methylsulfonyl)phenyl]acetamide.

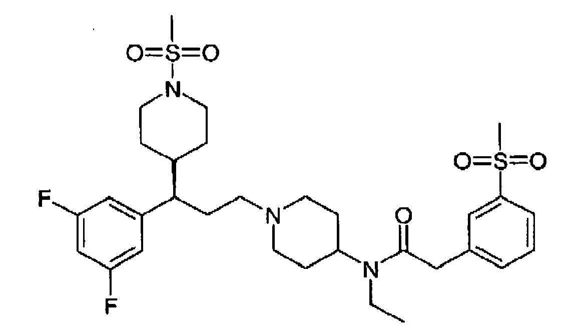

3-(Methylsulfonyl)phenylacetic acid (96 mg) was disolved in dichloromethane (5 ml) and carbonyldiimidazole (73 mg) added. The reaction mixture was stirred at room temperature for 3hours. *N*-(1-{(3*R*)-3-(3,5-Difluorophenyl)-3-[1-(methylsulfonyl)piperidin-4-yl]propyl}piperidin-4-yl)-*N*-ethylamine (200 mg) in dichloromethane (5 ml) was added and the reaction mixture was allowed to stand at room temperature for 72 hours. PS isocyanate resin (1. mm/g) (0.5 g) was added and the reaction mixture stirred at room temperature for 2hours, then filtered and evaporated. The residue was purified by column chromatography eluting with ethyl acetate-20%methanol/ethyl acetate to yield the title compound as a foam (105 mg).

NMR CDCl₃: 1.0-2.1 (m, 21H), 2.3-2.6 (m, 3H), 2.65 (s, 3H), 2.9 (s, 3H), 3.3 (m, 2H), 3.65 (d, 1H), 3.7 (m, 2H), 3.75 (d, 1H), 6.6 (m, 3H), 7.5 (m, 2H), 7.75 (m, 2H).

### Method A

### Preparation of (3R)-3-(3,5-difluorophenyl)-3-[1-(methylsulfonyl)piperidin-4-yl]propanal

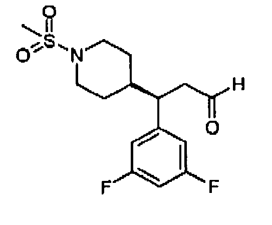

### Step 1 Preparation of (2E)-3-[1-(methylsulfonyl)piperidin-4-yl]acryloyl chloride.

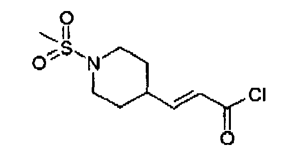

Oxalyl chloride (5.1 g) was added to a solution of (2*E*)-3-[1-(methylsulfonyl)piperidin-4-yl]acrylic acid (9.4g) in dichloromethane containing 2-3 drops of DMF and the mixture was stirred at room temperature for 1.5 hours. The reaction mixture was evaporated to dryness and the residue obtained was used directly in the next step.

### Step 2 Preparation of (4R,5S)-1,5-dimethyl-3-{(2E)-3-[1-(methylsulfonyl)piperidin-4-yl]prop-2-enoyl}-4-phenylimidazolidin-2-one.

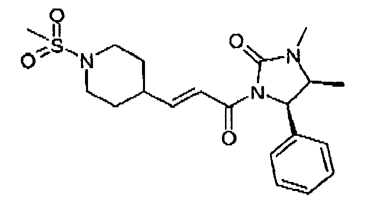

Lithium bis(trimethylsilyl)amide (8 ml of a 1M solution in THF) was added dropwise to a suspension of (4R,5S)-1,5-dimethyl-4-phenyl-2-imidazolidinone (1.52g) in THF (20 ml) under argon at -10°C. The reaction mixture was stirred at -10°C for 10 minutes, allowed to warm to 0°C and maintained at this temperature for 10 minutes then cooled again to -10°C. The solution of the acid chloride (2g dissolved in 10 ml of dichloromethane) prepared in Step 1 was added dropwise and the reaction mixture was allowed to warm to room temperature and washed with water (100 ml). The aqueous extract was extracted with ethyl acetate (3x50 ml) and the ethyl acetate extracts were dried and the residue passed through a 90g Biotage column eluting with a solvent gradient (50% ethyl acetate/isohexane - 70% ethyl acetate/isohexane). Yield 1.89g, LC-MS MH⁺ 406, NMR (CDCl₃): 0.8 (d, 3H), 1.5-1.6 (m, 3H), 1.9 (m, 2H), 2.3 (m, 1H), 2.7 (m, 2H), 2.75 (s, 3H), 2.8 (s, 3H), 3.75 (m, 2H), 3.9 (m, 1H), 5.3 (d, 1H), 6.85 (d-d, 1H), 7.1 (d, 1H), 7.2-7.35 (m, 3H), 7.45 (d, 1H).

### Step 3 Preparation of (4S,5R)-1-{(3R)-3-(3,5-difluorophenyl)-3-[1-(methylsulfonyl)piperidin-4-yl]propanoyl}-3,4-dimethyl-5-phenylimidazolidin-2-one.

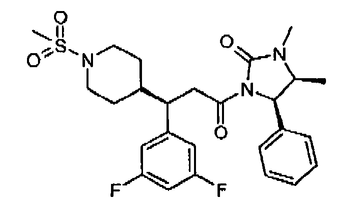

### Step A

TMEDA (11.6g) was added to a suspension of copper iodide (19.4g) in THF (240 ml) under argon and the mixture was stirred for 45 minutes then cooled to -70°C. A solution of 3,5-difluorophenyl magnesium bromide in THF (201.1 ml of a 0.5M solution in THF) was added over 10 minutes and the mixture was stirred at -70°C for 30 minutes.

### Step B

Di-n-butylboron triflate (100.7 ml of 1M solution in dichloromethane) was added to a suspension of (4*R*,5*S*)-1,5-dimethyl-3-{(2*E*)-3-[1-(methylsulfonyl)piperidin-4-yl]prop-2-enoyl}-4-phenylimidazolidin-2-one (20.41g) [Step 2] in THF maintained at -40°C and stirring was continued for 10 minutes and the mixture was cooled to -70°C and added via a cannula to the cuprate suspension prepared in step A. The reaction mixture was stirred at -70°C for 1 hour and allowed to warm to room temperature, then saturated ammonium chloride solution (200 ml) was added. The THF was evaporated and ethyl acetate (200 ml) was added. Air was blown through this mixture for 1 hour. The ethyl acetate layer was collected and the aqueous portion was extracted with ethyl acetate (2x100 ml). The combined ethyl acetate extracts were washed with saturated ammonium chloride solution (2x00 ml), dried and evaporated to dryness. The residue was purified by chromatography on silica eluting with a solvent gradient of ethyl acetate-isohexane (1:1) to neat ethyl acetate to give the title compound as a white solid, yield 25g, NMR (CDCl₃) 0.78 (d, 3H), 1.2-1.6 (m, 6H), 1.9 (m, 1H), 2.4-2.65 (m, 2H), 2.75 (s, 3H), 2.85 (s, 3H), 3-3.2 (m, 2H), 3.7-3.9 (m, 4H), 5.2 (d, 1H), 6.6(m, 3H), 6.85 (m, 2H), 7.2 (m, 3H).

### Step 4 Preparation of (3R)-3-(3,5-difluorophenyl)-3-[1-(methylsulfonyl)piperidin-4-yl]propan-1-ol

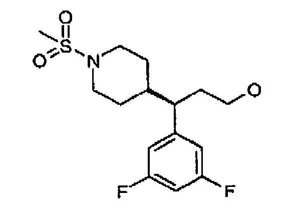

Lithium borohydride (48 ml of 2M solution in THF) was added to a solution of (4*S*,5*R*)-1-{(3*R*)-3-(3,5-difluorophenyl)-3-[1-(methylsulfonyl)piperidin-4-yl]propanoyl }-3,4-dimethyl-5-phenylimidazolidin-2-one (25g) in THF (200 ml) and the mixture was heated at 70°C for 3 hours then allowed to cool to room temperature and stirring was continued for 16 hours. Ethanol was added carefully (20 ml) and the reaction mixture was acidified to pH 4 by addition of 2M HCl. The THF was evaporated and the residue dissolved in dichloromethane (100 ml) and this was washed with water (100 ml) and dried. The solvent was removed and the product was purified by chromatography on a Biotage 65 column eluted with a 1:1 mixture of ethyl acetate/isohexane. Yield 13g, NMR (CDCl₃): 1.2-1.8 (m, 5H), 1.95-2.2 (m, 2H), 2.5-2.7 (m, 3H), 2.75 (s, 3H), 3.3-3.6 (m, 2H), 3.7 -3.9 (m, 2H), 6.65 (m, 3H).

### Step 5 Preparation of title compound.

Dess-Martin periodinane (5.09g) was added to a solution of (R) 3-(N-methanesulphonylpiperidin-4-yl)-3-(3,5-difluorophenyl)propanol (4.0g) in dichloromethane (100 ml) and the mixture was stirred for 1.5 hours. The reaction mixture was washed with 2M NaOH (2x100 ml) and dried. The solution of the title compound in dichloromethane was used in subsequent reactions.

### Method B

### N-ethyl-2-[4-(methylsulfonyl)phenyl]-N-piperidin-4-ylacetamide

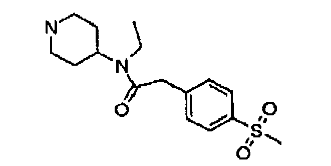

### Step 1: Preparation of 1-phenylmethyl-4-ethylaminopiperidine dihydrochloride

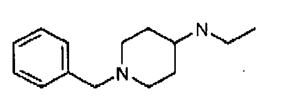

To a solution of 1-phenylmethyl-4-piperidone (25.0g, 132mmol) in THF (250mL) was added ethylamine hydrochloride (12.0g, 147 mol) and methanol (50mL) and the resulting mixture stirred at room temperature for 10min. Sodium triacetoxyborohydride (40g, 189mmol) was added portionwise and the resulting mixture stirred at room temperature for 1h. 2M Sodium hydroxide solution (250mL) was added and the resulting mixture extracted with diethyl ether. The organic extracts were dried (K₂CO₃) and evaporated to give 1-phenylmethyl-4-ethylammopiperidine as an oil. This was dissolved in ethanol (500mL) and concentrated hydrochloric acid (20mL) was added. The resulting crystals were collected, washed with diethyl ether and dried yield (38 g); NMR: (CDCl₃): 1.10 (t, 3H), 1.40 (m, 2H), 1.83 (m, 2H), 2.02 (m, 2H), 2.65 (q, 2H), 2.85 (m, 2H), 3.50 (s, 2H), 3.75 (m, 1H), 7.2 - 7.4 (m, 5H); MS: 219 (MH+).

### Step 2: Preparation of N-(1-benzylpiperidin-4-yl)-N-ethyl-2-[4-(methylsulfonyl)phenyl]acetamide.

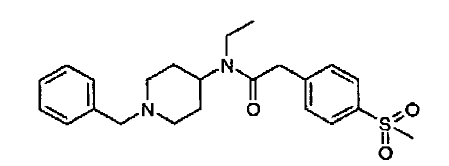

To a solution of 1-phenylmethyl-4-ethylaminopiperidine dihydrochloride (32.0g, 110mmol) in DCM (500mL) was added *N,N*-diisopropylethylamine (60mL) with stirring to ensure complete dissolution. 4-Methanesulfonylphenylacetic acid (25.0g, 117mmol), 4-dimethylaminopyridine (2.0g) and dicyclohexylcarbodiimide (25.0g, 121mmol) were added and the resulting mixture was stirred at room temperature for 20h. The precipitate was removed by filtration and the resulting solution was washed successively with 2N aqueous HCl, water and 1N aqueous NaOH, dried (MgSO₄) and evaporated. The residue was purified by silica gel chromatography (eluent: 10% MeOH/ethyl acetate) to afford the sub-titled compound (35 g, 76%); NMR: 1.00 and 1.14 (t, 3H), 1.45 and 1.70 (m, 2H), 1.95 (br m, 2H), 2.80 (br m, 2H), 3.18 (s, 3H), 3.20 and 3.33 (q, 2H), 3.45 (s, 2H), 3.80 and 3.87 (s, 2H), 3.70 and 4.10 (m, 1H), 7.2 - 7.3 (m, 5H), 7.48 (m, 2H), 7.82 (m, 2H); MS: 415 (MH+).

### Step 3: Preparation of the title compound

To a solution of *N*-(1-benzylpiperidin-4-yl)-*N*-ethyl-2-[4-(methylsulfonyl)phenyl]-acetamide (34g, 82mmol) in ethanol (600mL) was added ammonium formate (40g). The mixture was purged with argon and 30% Pd on carbon (4.2g) was added. The resulting mixture was stirred at reflux for 4h, then allowed to cool and filtered through diatomaceous earth. The filtrate was evaporated to give a thick oil which solidified on standing to yield the title compound (24.9g, 94%); NMR: 1.02 and 1.15 (t, 3H), 1.4-1.6 (br m, 4H), 2.45 (m, 2H), 2.93 (br m, 2H), 3.18 (s, 3H), 3.20 and 3.32 (q, 2H), 3.72 and 4.18 (m, 1H), 3.80 and 3.87 (s, 2H), 7.50 (m, 2H), 7.85 (m, 2H); MS: 325 (MH+).

### EXAMPLE 5

The ability of compounds to inhibit the binding of RANTES was assessed by an *in vitro* radioligand binding assay. Membranes were prepared from Chinese hamster ovary cells which expressed the recombinant human CCR5 receptor. These membranes were incubated with 0.1 nM iodinated RANTES, scintillation proximity beads and various concentrations of the compounds of the invention in 96-well plates. The amount of iodinated RANTES bound to the receptor was determined by scintillation counting. Competition curves were obtained for compounds and the concentration of compound which displaced 50% of bound iodinated RANTES was calculated (IC₅₀). Compounds of formula (I) having an IC₅₀ of less than 50µM form a further aspect of the invention.

### EXAMPLE 6

The ability of compounds to inhibit the binding of MIP-1α was assessed by an *in vitro* radioligand binding assay. Membranes were prepared from Chinese hamster ovary cells which expressed the recombinant human CCR5 receptor. These membranes were incubated with 0.1nM iodinated MIP-1α, scintillation proximity beads and various concentrations of the compounds of the invention in 96-well plates. The amount of iodinated MIP-1α bound to the receptor was determined by scintillation counting. Competition curves were obtained for compounds and the concentration of compound which displaced 50% of bound iodinated MIP-1α was calculated (IC₅₀). Compounds of formula (I) having an IC₅₀ of less than 50µM form yet another aspect of the invention.

Results from this test for certain compounds of the invention are presented in Table II. In Table II the results are presented as Pic50 values. A Pic50 value is the negative log (to base 10) of the IC₅₀ result, so an IC50 of 1µM (that is 1 x 10⁻⁶M) gives a Pic50 of 6. If a compound was tested more than once then the data below is an average of the probative tests results.

**Table II**

| Compound No. | Table No | Pic50 |
|---|---|---|
| 1 | I | 9.5 |
| 2 | I | 9.3 |
| 3 | I | 8.45 |
| 4 | I | 8.8 |
| 5 | I | 9.4 |
| 6 | I | 9 |

## Claims

1. The compound *N*-(1-{(3*R*)-3-(3,5-difluorophenyl)-3-[1-(methylsulfonyl)piperidin-4-yl]propyl}piperidin-4-yl)-*N*-ethyl-2-[4-(methylsulfonyl)phenyl]-acetamide, or a pharmaceutically acceptable salt thereof.

2. The compound of claim 1 wherein the pharmaceutically acceptable salt is selected from hydrochloride, hydrobromide, phosphate, acetate, fumarate, maleate, succinate, tartrate, citrate, oxalate, methanesulphonate or *p*-toluenesulphonate.

3. The compound of claims 1 or 2 wherein the compound is in the form of the succinate salt.

4. The compound of claims 1 or 2 wherein the compound is in the form of the fumarate salt.

5. A process for preparing of a compound as claimed in claim 1, the process comprising reductive amination of (3*R*)-3-(3,5-difluorophenyl)-3-[1-(methylsulfonyl)piperidin-4-yl]propanal with *N*-ethyl-2-[4-(methylsulfonyl)phenyl]-*N*-piperidin-4-ylacetamide in the presence of NaBH(OAc)₃ (wherein Ac is C(O)CH₃) and acetic acid, in a suitable solvent at room temperature.

6. A pharmaceutical composition which comprises a compound as claimed in claims 1 to 4, or a pharmaceutically acceptable salt thereof, and a pharmaceutically acceptable adjuvant, diluent or carrier.

7. A compound as claimed in claims 1 to 4, or a pharmaceutically acceptable salt thereof, for use as a medicament.

8. A compound as claimed in claims 1 to 4, or a pharmaceutically acceptable salt thereof, in the manufacture of a medicament for use in therapy.

9. The use of a compound of the formula (I) as claimed in claims 1 to 4, or a pharmaceutically acceptable salt thereof, as a medicament for the treatment of transplant rejection, respiratory disease, psoriasis or rheumatoid arthritis.

10. A compound as claimed in claims 1 to 4, or a pharmaceutically acceptable salt thereof or solvate thereof for use as a medicament for the treatment of rheumatoid arthritis.

## Patentansprüche

1. Die Verbindung *N*-(1-{(3*R*)-3-(3,5-Difluorphenyl)-3-[1-(methylsulfonyl)piperidin-4-yl]propyl}-piperidin-4-yl)-*N*-ethyl-2-[4-(methylsulfonyl)-phenyl]acetamid oder ein pharmazeutisch annehmbares Salz davon.

2. Verbindung nach Anspruch 1, wobei das pharmazeutisch annehmbare Salz aus Hydrochlorid, Hydrobromid, Phosphat, Acetat, Fumarat, Maleat, Succinat, Tartrat, Citrat, Oxalat, Methansulfonat oder *p*-Toluolsulfonat ausgewählt ist.

3. Verbindung nach Anspruch 1 oder 2, wobei die Verbindung in Form des Succinatsalzes vorliegt.

4. Verbindung nach Anspruch 1 oder 2, wobei die Verbindung in Form des Fumaratsalzes vorliegt.

5. Verfahren zur Herstellung einer Verbindung nach Anspruch 1, bei dem man (3*R*)-3-(3,5-Difluorphenyl)-3-[1-(methylsulfonyl)piperidin-4-yl]-propanal in Gegenwart von NaBH(OAc)₃ (wobei Ac für C(O)CH₃ steht) und Essigsäure in einem geeigneten Lösungsmittel bei Raumtemperatur mit *N*-Ethyl-2-[(4-methylsulfonyl)phenyl]-*N*-piperidin-4-ylacet-amid reduktiv aminiert.

6. Pharmazeutische Zusammensetzung, die eine Verbindung nach den Ansprüchen 1 bis 4 oder ein pharmazeutisch annehmbares Salz davon und einen pharmazeutisch annehmbaren Hilfsstoff, ein pharmazeutisch annehmbares Verdünnungsmittel oder einen pharmazeutisch annehmbaren Träger enthält.

7. Verbindung nach den Ansprüchen 1 bis 4 oder ein pharmazeutisch annehmbares Salz davon zur Verwendung als Arzneimittel.

8. Verbindung nach den Ansprüchen 1 bis 4 oder ein pharmazeutisch annehmbares Salz davon bei der Herstellung eines Arzneimittels zur Verwendung bei der Therapie.

9. Verwendung einer Verbindung der Formel (I) nach den Ansprüchen 1 bis 4 oder eines pharmazeutisch annehmbaren Salzes davon als Arzneimittel zur Behandlung von Transplantatabstoßung, Atemwegserkrankung, Psoriasis oder rheumatoider Arthritis.

10. Verbindung nach den Ansprüchen 1 bis 4 oder ein pharmazeutisch annehmbares Salz davon oder ein Solvat davon zur Verwendung eines Arzneimittels zur Behandlung von rheumatoider Arthritis.

## Revendications

1. Composé *N*-(1-{(3*R*)-3-(3-5-difluorophényl)-3-[1-(méthylsulfonyl)pipéridin-4-yl]propyl}pipéridin-4-yl)-*N*-éthyl-2-[4-(méthylsulfonyl)phényl]-acétamide, ou un sel de qualité pharmaceutique dudit composé.

2. Composé conforme à la revendication 1, où le sel de qualité pharmaceutique est choisi parmi les suivants : chlorhydrate, bromhydrate, phosphate, acétate, fumarate, maléate, succinate, tartrate, citrate, oxalate, méthanesulfonate et *p-*toluènesulphonate.

3. Composé conforme aux revendications 1 ou 2, où le composé est sous forme du sel de succinate.

4. Composé conforme aux revendications 1 ou 2, où le composé est sous forme du sel de fumarate.

5. Procédé de synthèse d'un composé conforme à la revendication 1, ledit procédé comprenant l'amination réductrice du (3*R*)-3-(3, 5-difluorophényl)-3-[1-(méthylsulfonyl)pipéridin-4-yl]propanal par le *N*-éthyl-2-[4-(méthylsulfonyl)phényl]-*N*-pipéridin-4-ylacétamide en présence de NaBH(OAc)₃ (où Ac représente C(O)CH₃) et d'acide acétique, dans un solvant adapté à température ambiante.

6. Composition pharmaceutique comprenant un composé conforme aux revendications 1 à 4, ou l'un de ses sels de qualité pharmaceutique, et un adjuvant, diluant ou vecteur de qualité pharmaceutique.

7. Composé conforme aux revendications 1 à 4, ou l'un de ses sels de qualité pharmaceutique, pour emploi en tant que médicament.

8. Composé conforme aux revendications 1 à 4, ou l'un de ses sels de qualité pharmaceutique, dans la fabrication d'un médicament pour emploi thérapeutique.

9. Emploi d'un composé de formule (I) conforme aux revendications 1 à 4, ou sel de qualité pharmaceutique dudit composé, en tant que médicament dans le traitement d'un rejet de greffon, d'une pathologie respiratoire, du psoriasis ou de la polyarthrite rhumatoïde.

10. Composé conforme aux revendications 1 à 4, ou l'un de ses sels de qualité pharmaceutique ou l'un de leurs solvates, pour emploi en tant que médicament dans le traitement de la polyarthrite rhumatoïde.
